# EUROPEAN PATENT SPECIFICATION

(11) **EP 1 315 820 B1**
(45) Date of publication and mention of the grant of the patent: **14.09.2005**
(21) Application number: 01957975.4
(22) Date of filing: 17.07.2001
(51) Int. Cl.: C12N 15/60, C12N 15/77, C12N 1/21, C12P 13/08, C12P 13/12, C12N 9/88, C12Q 1/68, A23K 1/00

(54) **NUCLEOTIDE SEQUENCES WHICH CODE FOR S-ADENOSYL HOMOCYSTEINASE (SAHH)**
SAHH (S-ADENOSYL HOMOCYSTEINASE) KODIERENDE NUKLEOTIDSEQUENZEN
SEQUENCES NUCLEOTIDIQUES CODANT POUR LE GENE SAHH

(30) Priority: 09.09.2000 DE 10044706; 28.02.2001 DE 10109685
(43) Date of publication of application: 04.06.2003
(60) Divisional application: 04027613.1
(73) Proprietor: Degussa AG, 40474 Düsseldorf (DE)
(72) Inventor: FARWICK, Mike, 33615 Bielefeld (DE); HUTHMACHER, Klaus, 63571 Gelnhausen (DE); BREHME, Jennifer, 33649 Bielefeld (DE); PFEFFERLE, Walter, 33790 Halle (Westf.) (DE); BINDER, Michael, 33803 Steinhagen (Westf.) (DE); GREISSINGER, Dieter, 61194 Niddatal (DE); THIERBACH, Georg, 33613 Bielefeld (DE)
(86) International application number: PCT/EP2001/008222
(87) International publication number: WO 2002/020806

(56) References cited:
- EP-A- 0 287 152
- EP-A- 0 435 132
- EP-A- 1 108 790
- WO-A-01/00843
- WO-A-01/66573
- DE-A- 3 823 451
- US-A- 3 729 381
- DATABASE EMBL [Online] MTY20B11, ACC NO: Z95121, 6 May 1997 (1997-05-06) retrieved from EBI XP002183842
- EGGELING L ET AL: "IMPROVED L-LYSINE YIELD WITH CORYNEBACTERIUM GLUTAMICUM: USE OF DAPA RESULTING IN INCREASED FLUX COMBINED WITH GROWTH LIMITATION" APPLIED MICROBIOLOGY AND BIOTECHNOLOGY, SPRINGER VERLAG, BERLIN, DE, vol. 49, no. 1, 1998, pages 24-30, XP000918549 ISSN: 0175-7598
- SAHM H ET AL: "CONSTRUCTION OF L-LYSINE, L-THREONINE-, OR L-ISOLEUCINE-OVERPRODUCING STRAINS OF CORYNEBACTERIUM GLUTAMICUM" ANNALS OF THE NEW YORK ACADEMY OF SCIENCES, NEW YORK ACADEMY OF SCIENCES, NEW YORK, NY, US, vol. 782, 1996, pages 25-39, XP000943151 ISSN: 0077-8923

### SEQUENCE PROTOCOL

<110> Degussa AG
<120> Nucleotide sequences which code for the sahH gene
<130> 000493 BT
<140>
   <141>
<160> 4
<170> PatentIn Ver. 2.1
<210> 1
   <211> 1939
   <212> DNA
   <213> Corynebacterium glutamicum
<220>
   <221> CDS
   <222> (227)..(1720)
   <223> sahH gene
<400> 1
   tcgcggccat catgtttggc gctccatatt ctgcgggcca ggtgctggag aattacccat 60
   ttatccgcga actcgtcggt gagggctctg aagctcttcg cggtgctgca gcgcaggttt 120
   tggaagaggc agatgtggaa tatgacctcg aagcttattt agaggccctc aactagccct 180
   ccactaaaca gcttcaatca attcggtgtc cactccaaca tgtaga gtg gtg cgc    235
                                                    Met Val Arg
                                                      1
   gtt aaa aaa gtt ttc cta att ttc att ttc tta aaa gga gct cgc cag   283
   Val Lys Lys Val Phe Leu Ile Phe Ile Phe Leu Lys Gly Ala Arg Gln
       5                   10                  15
   gac atg gca cag gtt atg gac ttc aag gtt gcc gat ctt tca cta gca   331
   Asp Met Ala Gln Val Met Asp Phe Lys Val Ala Asp Leu Ser Leu Ala
    20                  25                  30                  35
   gag gca gga cgt cac cag att cgt ctt gca gag tat gag atg cca ggt   379
   Glu Ala Gly Arg His Gln Ile Arg Leu Ala Glu Tyr Glu Met Pro Gly
                   40                  45                  50
   ctc atg cag ttg cgc aag gaa ttc gca gac gag cag cct ttg aag ggc   427
   Leu Met Gln Leu Arg Lys Glu Phe Ala Asp Glu Gln Pro Leu Lys Gly
               55                  60                  65
   gcc cga att gct ggt tct atc cac atg acg gtc cag acc gcc gtg ctt   475
   Ala Arg Ile Ala Gly Ser Ile His Met Thr Val Gln Thr Ala Val Leu
           70                  75                  80
   att gag acc ctc act gct ttg ggc gct gag gtt cgt tgg gct tcc tgc   523
   Ile Glu Thr Leu Thr Ala Leu Gly Ala Glu Val Arg Trp Ala Ser Cys
       85                  90                  95
   aac att ttc tcc acc cag gat gag gct gca gcg gct atc gtt gtc ggc    571
   Asn Ile Phe Ser Thr Gln Asp Glu Ala Ala Ala Ala Ile Val Val Gly
   100                 105                 110                 115
   tcc ggc acc gtc gaa gag cca gct ggt gtt cca gta ttc gcg tgg aag    619
   Ser Gly Thr Val Glu Glu Pro Ala Gly Val Pro Val Phe Ala Trp Lys
                   120                 125                 130
   ggt gag tca ctg gag gag tac tgg tgg tgc atc aac cag atc ttc agc    667
   Gly Glu Ser Leu Glu Glu Tyr Trp Trp Cys Ile Asn Gln Ile Phe Ser
               135                 140                 145
   tgg ggc gat gag ctg cca aac atg atc ctc gac gac ggc ggt gac gcc    715
   Trp Gly Asp Glu Leu Pro Asn Met Ile Leu Asp Asp Gly Gly Asp Ala
           150                 155                 160
   acc atg gct gtt att cgc ggt cgc gaa tac gag cag gct ggt ctg gtt    763
   Thr Met Ala Val Ile Arg Gly Arg Glu Tyr Glu Gln Ala Gly Leu Val
       165                 170                 175
   cca cca gca gag gcc aac gat tcc gat gag tac atc gca ttc ttg ggc    811
   Pro Pro Ala Glu Ala Asn Asp Ser Asp Glu Tyr Ile Ala Phe Leu Gly
   180                 185                 190                 195
   atg ctg cgt gag gtt ctt gct gca gag cct ggc aag tgg ggc aag atc    859
   Met Leu Arg Glu Val Leu Ala Ala Glu Pro Gly Lys Trp Gly Lys Ile
                   200                 205                 210
   gct gag gcc gtt aag ggt gtc acc gag gaa acc acc acc ggt gtg cac    907
   Ala Glu Ala Val Lys Gly Val Thr Glu Glu Thr Thr Thr Gly Val His
               215                 220                 225
   cgc ctg tac cac ttc gct gaa gaa ggc gtg ctg cct ttc cca gcg atg    955
   Arg Leu Tyr His Phe Ala Glu Glu Gly Val Leu Pro Phe Pro Ala Met
           230                 235                 240
   aac gtc aac gac gct gtc acc aag tcc aag ttt gat aac aag tac ggc    1003
   Asn Val Asn Asp Ala Val Thr Lys Ser Lys Phe Asp Asn Lys Tyr Gly
       245                 250                 255
   acc cgc cac tcc ctg atc gac ggc atc aac cgc gcc act gac atg ctc    1051
   Thr Arg His Ser Leu Ile Asp Gly Ile Asn Arg Ala Thr Asp Met Leu
   260                 265                 270                 275
   atg ggc ggc aag aac gtg ctt gtc tgc ggt tac ggc gat gtc ggc aag    1099
   Met Gly Gly Lys Asn Val Leu Val Cys Gly Tyr Gly Asp Val Gly Lys
                   280                 285                 290
   ggc tgc gct gag gct ttc gac ggc cag ggc gct cgc gtc aag gtc acc    1147
   Gly Cys Ala Glu Ala Phe Asp Gly Gln Gly Ala Arg Val Lys Val Thr
               295                 300                 305
   gaa gct gac cca atc aac gct ctt cag gct ctg atg gat ggc tac tct    1195
   Glu Ala Asp Pro Ile Asn Ala Leu Gln Ala Leu Met Asp Gly Tyr Ser
           310                 315                 320
   gtg gtc acc gtt gat gag gcc atc gag gac gcc gac atc gtg atc acc   1243
   Val Val Thr Val Asp Glu Ala Ile Glu Asp Ala Asp Ile Val Ile Thr
       325                 330                 335
   gcg acc ggc aac aag gac atc att tcc ttc gag cag atg ctc aag atg   1291
   Ala Thr Gly Asn Lys Asp Ile Ile Ser Phe Glu Gln Met Leu Lys Met
   340                 345                 350                 355
   aag gat cac gct ctg ctg ggc aac atc ggt cac ttt gat aat gag atc   1339
   Lys Asp His Ala Leu Leu Gly Asn Ile Gly His Phe Asp Asn Glu Ile
                   360                 365                 370
   gat atg cat tcc ctg ttg cac cgc gac gac gtc acc cgc acc acg atc   1387
   Asp Met His Ser Leu Leu His Arg Asp Asp Val Thr Arg Thr Thr Ile
               375                 380                 385
   aag cca cag gtc gac gag ttc acc ttc tcc acc ggt cgc tcc atc atc   1435
   Lys Pro Gln Val Asp Glu Phe Thr Phe Ser Thr Gly Arg Ser Ile Ile
           390                 395                 400
   gtc ctg tcc gaa ggt cgc ctg ttg aac ctt ggc aac gcc acc gga cac   1483
   Val Leu Ser Glu Gly Arg Leu Leu Asn Leu Gly Asn Ala Thr Gly His
       405                 410                 415
   cca tca ttt gtc atg tcc aac tct ttc gcc gat cag acc att gcg cag   1531
   Pro Ser Phe Val Met Ser Asn Ser Phe Ala Asp Gln Thr Ile Ala Gln
   420                 425                 430                 435
   atc gaa ctg ttc caa aac gaa gga cag tac gag aac gag gtc tac cgt   1579
   Ile Glu Leu Phe Gln Asn Glu Gly Gln Tyr Glu Asn Glu Val Tyr Arg
                   440                 445                 450
   ctg cct aag gtt ctc gac gaa aag gtg gca cgc atc cac gtt gag gct   1627
   Leu Pro Lys Val Leu Asp Glu Lys Val Ala Arg Ile His Val Glu Ala
               455                 460                 465
   ctc ggc ggt cag ctc acc gaa ctg acc aag gag cag gct gag tac atc   1675
   Leu Gly Gly Gln Leu Thr Glu Leu Thr Lys Glu Gln Ala Glu Tyr Ile
           470                 475                 480
   ggc gtt gac gtt gca ggc cca ttc aag ccg gag cac tac cgc tac       1720
   Gly Val Asp Val Ala Gly Pro Phe Lys Pro Glu His Tyr Arg Tyr
       485                 490                 495
   taatgattgt cagcattgag ggaatcgacg gcgccggcaa aaacaccctg gtttcggcat 1780
   taacgcaggt tattgatgca aaagtccttg cattcccacg ttatgaaacc tcgattcacg 1840
   cccaattggc cgcggaagca ctccacggcc gcatgggcga cctcaccgac agcgcctacg 1900
   ccatggccac gcttttcgcc ctcgaccgcc acttcgcga                        1939
<210> 2
   <211> 498
   <212> PRT
   <213> Corynebacterium glutamicum
<400> 2
   Met Val Arg Val Lys Lys Val Phe Leu Ile Phe Ile Phe Leu Lys Gly
    1               5                  10                  15
   Ala Arg Gln Asp Met Ala Gln Val Met Asp Phe Lys Val Ala Asp Leu
               20                  25                  30
   Ser Leu Ala Glu Ala Gly Arg His Gln Ile Arg Leu Ala Glu Tyr Glu
           35                  40                  45
   Met Pro Gly Leu Met Gln Leu Arg Lys Glu Phe Ala Asp Glu Gln Pro
       50                  55                  60
   Leu Lys Gly Ala Arg Ile Ala Gly Ser Ile His Met Thr Val Gln Thr
   65                  70                  75                  80
   Ala Val Leu Ile Glu Thr Leu Thr Ala Leu Gly Ala Glu Val Arg Trp
                   85                  90                  95
   Ala Ser Cys Asn Ile Phe Ser Thr Gln Asp Glu Ala Ala Ala Ala Ile
               100                 105                 110
   Val Val Gly Ser Gly Thr Val Glu Glu Pro Ala Gly Val Pro Val Phe
           115                 120                 125
   Ala Trp Lys Gly Glu Ser Leu Glu Glu Tyr Trp Trp Cys Ile Asn Gln
       130                 135                 140
   Ile Phe Ser Trp Gly Asp Glu Leu Pro Asn Met Ile Leu Asp Asp Gly
   145                 150                 155                 160
   Gly Asp Ala Thr Met Ala Val Ile Arg Gly Arg Glu Tyr Glu Gln Ala
                   165                 170                 175
   Gly Leu Val Pro Pro Ala Glu Ala Asn Asp Ser Asp Glu Tyr Ile Ala
               180                 185                 190
   Phe Leu Gly Met Leu Arg Glu Val Leu Ala Ala Glu Pro Gly Lys Trp
           195                 200                 205
   Gly Lys Ile Ala Glu Ala Val Lys Gly Val Thr Glu Glu Thr Thr Thr
       210                 215                 220
   Gly Val His Arg Leu Tyr His Phe Ala Glu Glu Gly Val Leu Pro Phe
   225                 230                 235                 240
   Pro Ala Met Asn Val Asn Asp Ala Val Thr Lys Ser Lys Phe Asp Asn
                   245                 250                 255
   Lys Tyr Gly Thr Arg His Ser Leu Ile Asp Gly Ile Asn Arg Ala Thr
               260                 265                 270
   Asp Met Leu Met Gly Gly Lys Asn Val Leu Val Cys Gly Tyr Gly Asp
           275                 280                 285
   Val Gly Lys Gly Cys Ala Glu Ala Phe Asp Gly Gln Gly Ala Arg Val
       290                 295                 300
   Lys Val Thr Glu Ala Asp Pro Ile Asn Ala Leu Gln Ala Leu Met Asp
   305                 310                 315                 320
   Gly Tyr Ser Val Val Thr Val Asp Glu Ala Ile Glu Asp Ala Asp Ile
                   325                 330                 335
   Val Ile Thr Ala Thr Gly Asn Lys Asp Ile Ile Ser Phe Glu Gln Met
               340                 345                 350
   Leu Lys Met Lys Asp His Ala Leu Leu Gly Asn Ile Gly His Phe Asp
           355                 360                 365
   Asn Glu Ile Asp Met His Ser Leu Leu His Arg Asp Asp Val Thr Arg
       370                 375                 380
   Thr Thr Ile Lys Pro Gln Val Asp Glu Phe Thr Phe Ser Thr Gly Arg
   385                 390                 395                 400
   Ser Ile Ile Val Leu Ser Glu Gly Arg Leu Leu Asn Leu Gly Asn Ala
                   405                 410                 415
   Thr Gly His Pro Ser Phe Val Met Ser Asn Ser Phe Ala Asp Gln Thr
               420                 425                 430
   Ile Ala Gln Ile Glu Leu Phe Gln Asn Glu Gly Gln Tyr Glu Asn Glu
           435                 440                 445
   Val Tyr Arg Leu Pro Lys Val Leu Asp Glu Lys Val Ala Arg Ile His
       450                 455                 460
   Val Glu Ala Leu Gly Gly Gln Leu Thr Glu Leu Thr Lys Glu Gln Ala
   465                 470                 475                 480
   Glu Tyr Ile Gly Val Asp Val Ala Gly Pro Phe Lys Pro Glu His Tyr
                   485                 490                 495
   Arg Tyr
<210> 3
   <211> 28
   <212> DNA
   <213> Artificial sequence
<220>
   <223> Description of the artificial sequence:Primer sahHex1
<400> 3
   gtggtacctt cggtgtccac tccaacat                                   28
<210> 4
   <211> 28
   <212> DNA
   <213> Artificial sequence
<220>
   <223> Description of the artificial sequence: Primer sahHex2
<400> 4
   catctagata ggcgctgtcg gtgaggtc                                   28

## Description

### Field of the Invention

The invention provides a process for the fermentative preparation of amino acids using bacteria in which the sahH gene is enhanced.

### Prior Art

L-Amino acids, in particular L-lysine and L-methionine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and produce amino acids are obtained in this manner.

Methods of the recombinant DNA technique have also been employed for some years for improving the strain of Corynebacterium strains which produce L-amino acid, by amplifying individual amino acid biosynthesis genes and investigating the effect on the amino acid production.

### Object of the Invention

The inventors had the object of providing new measures for improved fermentative preparation of amino acids.

### Summary of the Invention

Where L-amino acids or amino acids are mentioned in the following, this means one or more amino acid, including their salts, chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-Lysine and L-methionine are particularly preferred.

When L-lysine or lysine are mentioned in the following, not only the bases but also the salts, such as e.g. lysine monohydrochloride or lysine sulfate, are meant by this.

When L-methionine or methionine are mentioned in the following, the salts, such as e.g. methionine hydrochloride or methionine sulfate are also meant by this.

The invention furthermore relates to a process for the fermentative preparation of L-lysine, using coryneform bacteria which in particular already produce amino acids and in which the nucleotide sequences which code for the sahH gene are enhanced, in particular over-expressed.

### Detailed Description of the Invention

Polynucleotides which comprise the sequences according to the invention are suitable as hybridization probes for RNA, cDNA and DNA, in order to isolate, in the full length, nucleic acids or polynucleotides or genes which code for adenosyl homocysteinase or to isolate those nucleic acids or polynucleotides or genes which have a high similarity of sequence with that of the sahH gene.

Polynucleotides which comprise the sequences according to the invention are furthermore suitable as primers with the aid of which DNA of genes which code for adenosyl homocysteinase can be prepared by the polymerase chain reaction (PCR).

Such oligonucleotides which serve as probes or primers comprise at least 30, preferably at least 20, very particularly preferably at least 15 successive nucleotides. Oligonucleotides which have a length of at least 40 or 50 nucleotides are also suitable. Oligonucleotides with a length of at least 100, 150, 200, 250 or 300 nucleotides are optionally also suitable.

"Isolated" means separated out of its natural environment.

"Polynucleotide" in general relates to polyribonucleotides and polydeoxyribonucleotides, it being possible for these to be non-modified RNA or DNA or modified RNA or DNA.

The polynucleotides according to the invention include a polynucleotide according to SEQ ID No. 1 or a fragment prepared therefrom and also those which are at least 70%, preferably at least 80% and in particular at least 90% to 95% identical to the polynucleotide according to SEQ ID No. 1 or a fragment prepared therefrom.

"Polypeptides" are understood as meaning peptides or proteins which comprise two or more amino acids bonded via peptide bonds.

The polypeptides according to the invention include a polypeptide according to SEQ ID No. 2, in particular those with the biological activity of adenosyl homocysteinase, and also those which are at least 70%, preferably at least 80% and in particular at least 90% to 95% identical to the polypeptide according to SEQ ID No. 2 and have the activity mentioned.

The term "enhancement" in this connection describes the increase in the intracellular activity of one or more enzymes (proteins) in a microorganism which are coded by the corresponding DNA, for example by increasing the number of copies of the gene or genes, using a potent promoter or using a gene or allele which codes for a corresponding enzyme (protein) having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on the starting microorganism.

The microorganisms which the present invention provides can produce L-amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 and
Brevibacterium divaricatum ATCC14020
and L-amino acid-producing mutants or strains prepared therefrom.

To isolate the sahH gene or also other genes of C. glutamicum, a gene library of this microorganism is first set up in Escherichia coli (E. coli). The setting up of gene libraries is described in generally known textbooks and handbooks. The textbook by Winnacker: Gene und Klone, Eine Einführung in die Gentechnologie (Verlag Chemie, Weinheim, Germany, 1990), or the handbook by Sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326) (1992)) in turn describe a gene library of C. glutamicum ATCC13032 using the cosmid pHC79 (Hohn and Collins, Gene 11, 291-298 (1980)).

To prepare a gene library of C. glutamicum in E. coli it is also possible to use plasmids such as pBR322 (Bolivar, Life Sciences, 25, 807-818 (1979)) or pUC9 (Vieira et al., 1982, Gene, 19:259-268). Suitable hosts are, in particular, those E. coli strains which are restriction- and recombination-defective. An example of these is the strain DH5αmcr, which has been described by Grant et al. (Proceedings of the National Academy of Sciences USA, 87 (1990) 4645-4649). The long DNA fragments cloned with the aid of cosmids can in turn be subcloned in the usual vectors suitable for sequencing and then sequenced, as is described e.g. by Sanger et al. (Proceedings of the National Academy of Sciences of the United States of America, 74:5463-5467, 1977).

The resulting DNA sequences can then be investigated with known algorithms or sequence analysis programs, such as e.g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The new DNA sequence of C. glutamicum which codes for the sahH gene and which, as SEQ ID No. 1, is a constituent of the present invention has been found. The amino acid sequence of the corresponding protein has furthermore been derived from the present DNA sequence by the methods described above. The resulting amino acid sequence of the sahH gene product is shown in SEQ ID No. 2.

Coding DNA sequences which result from SEQ ID No. 1 by the degeneracy of the genetic code are also a constituent of the invention. In the same way, DNA sequences which hybridize with SEQ ID No. 1 or parts of SEQ ID No. 1 are a constituent of the invention. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i.e. are of neutral function. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth et al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2 are also a constituent of the invention.

In the same way, DNA sequences which hybridize with SEQ ID No. 1 or parts of SEQ ID No. 1 are a constituent of the invention. Finally, DNA sequences which are prepared by the polymerase chain reaction (PCR) using primers which result from SEQ ID No. 1 are a constituent of the invention. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology (1991) 41: 255-260). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50°C - 68°C, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50°C - 68°C being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise from 50°C to 68°C in steps of approx. 1 - 2°C. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce amino acids in an improved manner after over-expression of the sahH gene.

To achieve an over-expression, the number of copies of the corresponding genes can be increased, or the promoter and regulation region or the ribosome binding site upstream of the structural gene can be mutated. Expression cassettes which are incorporated upstream of the structural gene act in the same way. By inducible promoters, it is additionally possible to increase the expression in the course of fermentative amino acid production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome. Alternatively, an over-expression of the genes in question can furthermore be achieved by changing the composition of the media and the culture procedure.

Instructions in this context can be found by the expert, inter alia, in Martin et al. (Bio/Technology 5, 137-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in European Patent Specification 0 472 869, in US Patent 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in Patent Application WO 96/15246, in Malumbres et al. (Gene 134, 15 - 24 (1993)), in Japanese Laid-Open Specification JP-A-10-229891, in Jensen and Hammer (Biotechnology and Bioengineering 58, 191-195 (1998)), in Makrides (Microbiological Reviews 60:512-538 (1996)) and in known textbooks of genetics and molecular biology.

By way of example, for enhancement the sahH gene according to the invention was over-expressed with the aid of episomal plasmids. Suitable plasmids are those which are replicated in coryneform bacteria. Numerous known plasmid vectors, such as e.g. pZ1 (Menkel et al., Applied and Environmental Microbiology (1989) 64: 549-554), pEKEx1 (Eikmanns et al., Gene 102:93-98 (1991)) or pHS2-1 (Sonnen et al., Gene 107:69-74 (1991)) are based on the cryptic plasmids pHM1519, pBL1 or pGA1. Other plasmid vectors, such as e.g. those based on pCG4 (US-A 4,489,160), or pNG2 (Serwold-Davis et al., FEMS Microbiology Letters 66, 119-124 (1990)), or pAG1 (US-A 5,158,891), can be used in the same manner.

Plasmid vectors which are furthermore suitable are also those with the aid of which the process of gene amplification by integration into the chromosome can be used, as has been described, for example, by Reinscheid et al. (Applied and Environmental Microbiology 60, 126-132 (1994)) for duplication or amplification of the hom-thrB operon. In this method, the complete gene is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pGEM-T (Promega corporation, Madison, WI, USA), pCR2.1-TOPO (Shuman (1994). Journal of Biological Chemistry 269:32678-84; US-A 5,487,993), pCR®Blunt (Invitrogen, Groningen, Holland; Bernard et al., Journal of Molecular Biology, 234: 534-541 (1993)), pEM1 (Schrumpf et al, 1991, Journal of Bacteriology 173:4510-4516) or pBGSB (Spratt et al.,1986, Gene 41: 337-342). The plasmid vector which contains the gene to be amplified is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross over" event, the resulting strain contains at least two copies of the gene in question.

In addition, it may be advantageous for the production of L-amino acids to enhance, in particular over-express, one or more enzymes of the particular biosynthesis pathway, of glycolysis, of anaplerosis, of the citric acid cycle, of the pentose phosphate cycle, of amino acid export and optionally regulatory proteins, in addition to the sahH gene.

Thus, for the preparation of L-amino acids, in addition to enhancement of the sahH gene, one or more genes chosen from the group consisting of
- the dapA gene which codes for dihydrodipicolinate synthase (EP-B 0 197 335),
- the gap gene which codes for glyceraldehyde 3-phosphate dehydrogenase (Eikmanns (1992), Journal of Bacteriology 174:6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661),
- the pyc gene which codes for pyruvate carboxylase (DE-A-198 31 609),
- the mqo gene which codes for malate-quinone oxidoreductase (Molenaar et al., European Journal of Biochemistry 254, 395-403 (1998)),
- the lysC gene which codes for a feed-back resistant aspartate kinase (Accession No.P26512),
- the lysE gene which codes for lysine export (DE-A-195 48 222),
- the hom gene which codes for homoserine dehydrogenase (EP-A 0131171),
- the ilvA gene which codes for threonine dehydratase (Möckel et al., Journal of Bacteriology (1992) 8065-8072)) or the ilvA(Fbr) allele which codes for a "feed back resistant" threonine dehydratase (Möckel et al., (1994) Molecular Microbiology 13: 833-842),
- the ilvBN gene which codes for acetohydroxy-acid synthase (EP-B 0356739),
- the ilvD gene which codes for dihydroxy-acid dehydratase (Sahm and Eggeling (1999) Applied and Environmental Microbiology 65: 1973-1979),
- the zwa1 gene which codes for the Zwa1 protein (DE: 19959328.0, DSM 13115),
can be enhanced, in particular over-expressed.

It may furthermore be advantageous for the production of L-amino acids, in addition to the enhancement of the sahH gene, for one or more genes chosen from the group consisting of:
- the pck gene which codes for phosphoenol pyruvate carboxykinase (DE 199 50 409.1; DSM 13047)
- the pgi gene which codes for glucose 6-phosphate isomerase (US 09/396,478; DSM 12969),
- the poxB gene which codes for pyruvate oxidase (DE: 1995 1975.7; DSM 13114),
- the zwa2 gene which codes for the Zwa2 protein (DE: 19959327.2, DSM 13113)
to be attenuated, in particular for the expression thereof to be reduced.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

By attenuation measures, the activity or concentration of the corresponding protein is in general reduced to 0 to 50%, 0 to 25%, 0 to 10% or 0 to 5% of the activity or concentration of the wild-type protein.

In addition to over-expression of the sahH gene it may furthermore be advantageous for the production of amino acids to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of amino acids. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substance can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Organic and inorganic sulfur-containing compounds, such as, for example, sulfides, sulfites, sulfates and thiosulfates, can be used as a source of sulfur, in particular for the preparation of methionine.

Phosphoric acid, potassium dihydrogen phosphate or dipotassium hydrogen phosphate or the corresponding sodium-containing salts can be used as the source of phosphorus. The culture medium must furthermore comprise salts of metals, such as e. g. magnesium sulfate or iron sulfate, which are necessary for growth. Finally, essential growth substances, such as amino acids and vitamins, can be employed in addition to the above-mentioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of the desired product has formed. This target is usually reached within 10 hours to 160 hours.

The fermentation broths obtained in this way, in particular containing L-methionine, usually have a dry weight of 7.5 to 25 wt.% and contain L-methionine. It is furthermore also advantageous if the fermentation is conducted in a sugar-limited procedure at least at the end, but in particular over at least 30% of the duration of the fermentation. That is to say, the concentration of utilizable sugar in the fermentation medium is reduced to ≥ 0 to 3 g/l during this period.

The fermentation broth prepared in this manner, in particular containing L-methionine, is then further processed. Depending on requirements, all or some of the biomass can be removed from the fermentation broth by separation methods, such as e.g. centrifugation, filtration, decanting or a combination thereof, or left completely in it. This broth is then thickened or concentrated by known methods, such as e.g. with the aid of a rotary evaporator, thin film evaporator, falling film evaporator, by reverse osmosis, or by nanofiltration. This concentrated fermentation broth can then be worked up by methods of freeze drying, spray drying, spray granulation or by other processes to give a preferably free-flowing, finely divided powder.

This free-flowing, finely divided powder can then in turn by converted by suitable compacting or granulating processes into a coarse-grained, readily free-flowing, storable and largely dust-free product. In the granulation or compacting it is advantageous to employ conventional organic or inorganic auxiliary substances or carriers, such as starch, gelatin, cellulose derivatives or similar substances, such as are conventionally used as binders, gelling agents or thickeners in foodstuffs or feedstuffs processing, or further substances, such as, for example, silicas, silicates or stearates.

"Free-flowing" is understood as meaning powders which flow unimpeded out of the vessel with the opening of 5 mm (millimeters) of a series of glass outflow vessels with outflow openings of various sizes (Klein, Seifen, Ole, Fette, Wachse 94, 12 (1968)).

As described here, "finely divided" means a powder with a predominant content (> 50 %) with a particle size of 20 to 200 µm diameter. "Coarse-grained" means products with a predominant content (> 50 %) with a particle size of 200 to 2000 µm diameter. In this context, "dust-free" means that the product contains only small contents (< 5%) with particle sizes of less than 20 µm diameter. The particle size determination can be carried out with methods of laser diffraction spectrometry. The corresponding methods are described in the textbook on "Teilchengrößenmessung in der Laborpraxis" by R. H. Müller and R. Schuhmann, Wissenschaftliche Verlagsgesellschaft Stuttgart (1996) or in the textbook "Introduction to Particle Technology" by M. Rhodes, Verlag Wiley & Sons (1998).

"Storable" in the context of this invention means a product which can be stored for up to 120 days, preferably up to 52 weeks, particularly preferably 60 months, without a substantial loss (< 5%) of methionine occurring.

Alternatively, however, the product can be absorbed on to an organic or inorganic carrier substance which is known and conventional in feedstuffs processing, such as, for example, silicas, silicates, grits, brans, meals, starches, sugars or others, and/or mixed and stabilized with conventional thickeners or binders. Use examples and processes in this context are described in the literature (Die Mühle + Mischfuttertechnik 132 (1995) 49, page 817).

Finally, the product can be brought into a state in which it is stable to digestion by animal stomachs, in particular the stomach of ruminants, by coating processes ("coating") using film-forming agents, such as, for example, metal carbonates, silicas, silicates, alginates, stearates, starches, gums and cellulose ethers, as described in DE-C-4100920.

If the biomass is separated off during the process, further inorganic solids, for example added during the fermentation, are in general removed. In addition, the animal feedstuffs additive according to the invention comprises at least the predominant proportion of the further substances, in particular organic substances, which are formed or added and are present in solution in the fermentation broth, where these have not been separated off by suitable processes.

In one aspect of the invention, the biomass can be separated off to the extent of up to 70%, preferably up to 80&, preferably up to 90%, preferably up to 95%, and particularly preferably up to 100%. In another aspect of the invention, up to 20% of the biomass, preferably up to 15%, preferably up to 10%, preferably up to 5%, particularly preferably no biomass is separated off.

These organic substances include organic by-products which are optionally produced, in addition to the L-methionine, and optionally discharged by the microorganisms employed in the fermentation. These include L-amino acids chosen from the groups consisting of L-lysine, L-valine, L-threonine, L-alanine or L-tryptophan. They include vitamins chosen from the group consisting of vitamin B1 (thiamine), vitamin B2 (riboflavin),vitamin B5 (pantothenic acid), vitamin B6 (pyridoxine), vitamin B12 (cyanocobalamin), nicotinic acid/nicotinamide and vitamin E (tocopherol). They include furthermore organic acids which carry one to three carboxyl groups, such as, for example, acetic acid, lactic acid, citric acid, malic acid or fumaric acid. Finally, they also include sugars, such as, for example, trehalose. These compounds are optionally desired if they improve the nutritional value of the product.

These organic substances, including L-methionine and/or D-methionine and/or the racemic mixture D,L-methionine, can also be added, depending on requirements, as a concentrate or pure substance in solid or liquid form during a suitable process step. These organic substances mentioned can be added individually or as mixtures to the resulting or concentrated fermentation broth, or also during the drying or granulation process. It is likewise possible to add an organic substance or a mixture of several organic substances to the fermentation broth and a further organic substance or a further mixture of several organic substances during a later process step, for example granulation.

The product described above is suitable as a feedstuffs additive, i.e. feed additive, for animal nutrition.

The L-methionine content of the animal feedstuffs additive is conventionally 1 wt.% to 80 wt.%, preferably 2 wt.% to 80 wt.%, particularly preferably 4 wt.% to 80 wt.%, and very particularly preferably 8 wt.% to 80 wt.%, based on the dry weight of the animal feedstuffs additive. Contents of 1 wt.% to 60 wt.%, 2 wt.% to 60 wt.%, 4 wt.% to 60 wt.%, 6 wt.% to 60 wt.%, 1 wt.% to 40 wt.%, 2 wt.% to 40 wt.% or 4 wt.% to 40 wt.% are likewise possible. The water content of the feedstuffs additive is conventionally up to 5 wt.%, preferably up to 4 wt.%, and particularly preferably less than 2 wt.%.

The invention accordingly also provides a process for the preparation of an L-methionine-containing animal feedstuffs additive from fermentation broths, which comprises the steps
a) culture and fermentation of an L-methionine-producing microorganism in a fermentation medium;
b) removal of water from the L-methionine-containing fermentation broth (concentration) ;
c) removal of an amount of 0 to 100 wt.% of the biomass formed during the fermentation; and
d) drying of the fermentation broth obtained according to a) and/or b) to obtain the animal feedstuffs additive in the desired powder or granule form.
   If desired, one or more of the following steps can furthermore be carried out in the process according' to the invention:
e) addition of one or more organic substances, including L-methionine and/or D-methionine and/or the racemic mixture D,L-methionine, to the products obtained according to a), b) and/or c);
f) addition of auxiliary substances chosen from the group consisting of silicas, silicates, stearates, grits and bran to the substances obtained according to a) to d) for stabilization and to increase the storability; or
g) conversion of the substances obtained according to a) to e) into a form stable to an animal stomach, in particular rumen, by coating with film-forming agents.

Methods for the determination of L-amino acids are known from the prior art. The analysis can thus be carried out, for example, as described by Spackman et al. (Analytical Chemistry, 30, (1958), 1190) by ion exchange chromatography with subsequent ninhydrin derivation, or it can be carried out by reversed phase HPLC, for example as described by Lindroth et al. (Analytical Chemistry (1979) 51: 1167-1174).

The process according to the invention is used for fermentative preparation of amino acids.

The present invention is explained in more detail in the following with the aid of embodiment examples.

The following microorganism was deposited as a pure culture on 18th May 2001 at the Deutsche Sammlung für Mikroorganismen und Zellkulturen (DSMZ = German Collection of Microorganisms and Cell Cultures, Braunschweig, Germany) in accordance with the Budapest Treaty:

Escherichia coli DH5αmcr/pEC-XK99sahHa1ex as DSM 14316.

The isolation of plasmid DNA from Escherichia coli and all techniques of restriction, Klenow and alkaline phosphatase treatment were carried out by the method of Sambrook et al. (Molecular Cloning. A Laboratory Manual (1989) Cold Spring Harbor Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from Corynebacterium glutamicum ATCC 13032

Chromosomal DNA from Corynebacterium glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Diagnostics GmbH, Mannheim, Germany, Product Description SAP, Code no. 1758250). The DNA of the cosmid vector SuperCos1 (Wahl et al. (1987) Proceedings of the National Academy of Sciences USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Research 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) with 100 mg/l ampicillin. After incubation overnight at 37°C, recombinant individual clones were selected.

### Example 2

### Isolation and sequencing of the sahH gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Diagnostics GmbH, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, Holland, Product Description Zero Background Cloning Kit, Product No. K2500-01), was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5αMCR (Grant, 1990, Proceedings of the National Academy of Sciences U.S.A., 87:4645-4649) and plated out on LB agar (Lennox, 1955, Virology, 1:190) with 50 mg/l zeocin.

The plasmid preparation of the recombinant clones was carried out with Biorobot 9600 (Product No. 900200, Qiagen, Hilden, Germany). The sequencing was carried out by the dideoxy chain termination method of Sanger et al. (1977, Proceedings of the National Academy of Sciences U.S.A., 74:5463-5467) with modifications according to Zimmermann et al. (1990, Nucleic Acids Research, 18:1067). The "RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig. The computer-assisted coding region analysis was prepared with the XNIP program (Staden, 1986, Nucleic Acids Research, 14:217-231).

The resulting nucleotide sequence is shown in SEQ ID No. 1. Analysis of the nucleotide sequence showed an open reading frame of 1497 base pairs, which was called the sahH gene. The sahH gene codes for a protein of 498 amino acids.

### Example 3

### Preparation of a shuttle expression vector pEC-XK99EsahHex for enhancement of the sahH gene in C. glutamicum

### 3.1 Cloning of the sahH gene

From the strain ATCC 13032, chromosomal DNA is isolated by the method of Eikmanns et al. (Microbiology 140: 1817 -1828 (1994)). On the basis of the sequence of the sahH gene known for C. glutamicum from example 2, the following oligonucleotides were chosen for the polymerase chain reaction(see SEQ ID No. 3 and SEQ ID No. 4):

The primers shown were synthesized by MWG-Biotech AG (Ebersberg, Germany) and the PCR reaction was carried out by the standard PCR method of Innis et al. (PCR protocols. A guide to methods and applications, 1990, Academic Press) with Pwo-Polymerase from Roche Diagnostics GmbH (Mannheim, Germany). With the aid of the polymerase chain reaction, the primers allow amplification of a DNA fragment approx. 1.7 kb in size, which carries the sahH gene. Furthermore, the primer sahHex1 contains the sequence for the cleavage site of the restriction endonuclease Kpn1, and the primer sahHex2 the cleavage site of the restriction endonuclease XbaI, which are marked by underlining in the nucleotide sequence shown above.

The sahH fragment approx. 1.7 kb in size was cleaved with the restriction endonucleases KpnI and XbaI and then isolated from the agarose gel with the QiaExII Gel Extraction Kit (Product No. 20021, Qiagen, Hilden, Germany).

### 3.2 Construction of the shuttle vector pEC-XK99E

The E. coli - C. glutamicum shuttle vector pEC-XK99E was constructed according to the prior art. The vector contains the replication region rep of the plasmid pGA1 including the replication effector per (US-A- 5,175,108; Nesvera et al., Journal of Bacteriology 179, 1525-1532 (1997)), the kanamycin resistance gene aph(3')-IIa from Escherichia coli (Beck et al. (1982), Gene 19: 327-336), the replication origin of the trc promoter, the termination regions T1 and T2, the lacI^{q} gene (repressor of the lac operon of E. coli) and a multiple cloning site (mcs) (Norrander, J.M. et al. Gene 26, 101-106 (1983)) of the plasmid pTRC99A (Amann et al. (1988), Gene 69: 301-315).

The E. coli - C. glutamicum shuttle vector pEC-XK99E constructed was transferred into C. glutamicum DSM5715 by means of electroporation (Liebl et al., 1989, FEMS Microbiology Letters, 53:299-303). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from a transformant by conventional methods (Peters-Wendisch et al., 1998, Microbiology, 144, 915 -927), cleaved with the restriction endonuclease HindIII, and the plasmid was checked by subsequent agarose gel electrophoresis.

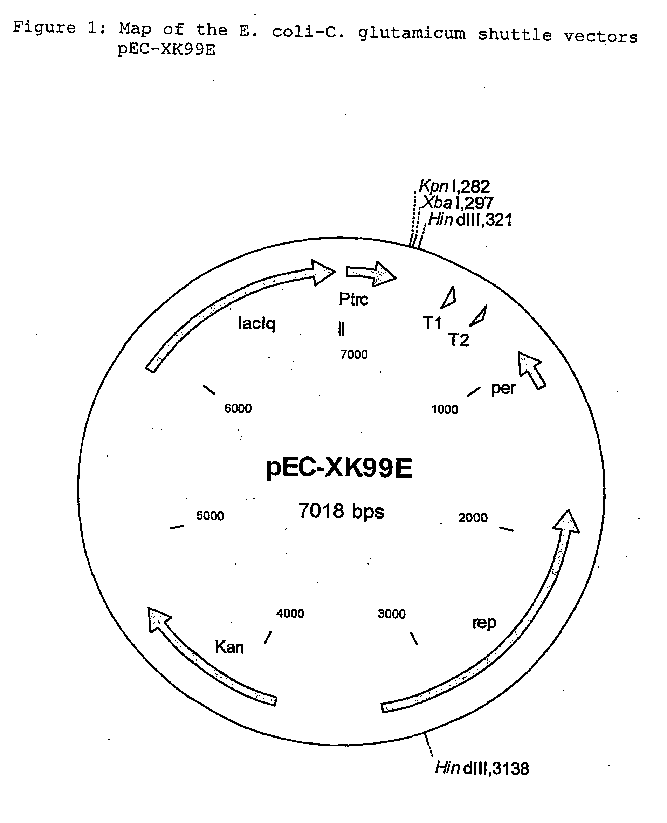

The plasmid construct obtained in this way was called pEC-XK99E (figure 1). The strain obtained by electroporation of the plasmid pEC-XK99E in the C. glutamicum strain DSM5715 was called DSM5715/pEC-XK99E and deposited as DSM13455 at the Deutsche Sammlung für Mikroorganismen und Zellkulturen (DSMZ = German Collection of Microorganisms and Cell Cultures, Braunschweig, Germany) in accordance with the Budapest Treaty.

### 3.3 Cloning of sahH in the E. coli-C. glutamicum shuttle vector pEC-XK99E

The E. coli - C. glutamicum shuttle vector pEC-XK99E described in example 3.2 was used as the vector. DNA of this plasmid was cleaved completely with the restriction enzymes KpnI and XbaI and then dephosphorylated with shrimp alkaline phosphatase (Roche Diagnostics GmbH, Mannheim, Germany, Product Description SAP, Product No. 1758250).

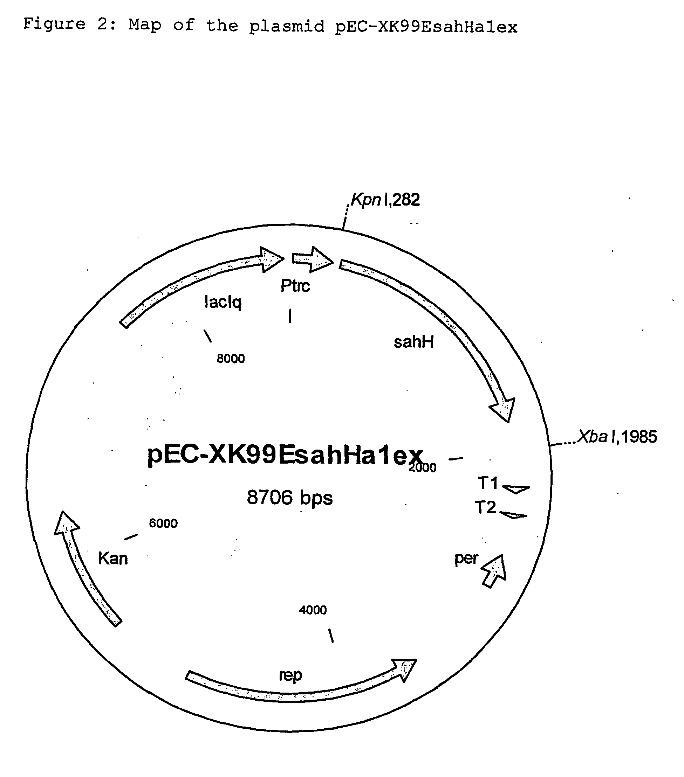

The sahH fragment 1.7 kb in size described in example 3.1, obtained by means of the PCR and restricted with the restriction endonucleases KpnI and XbaI was mixed with the vector pEC-XK99E prepared and the batch was treated with T4 DNA ligase (Amersham Pharmacia, Freiburg, Germany, Product Description T4-DNA-Ligase, Code no.27-0870-04). The ligation batch was transformed in the E. coli strain DH5αmcr (Hanahan, In: DNA cloning. A practical approach. Vol. I. IRL-Press, Oxford, Washington DC, USA). Selection of plasmid-carrying cells was made by plating out the transformation batch on LB agar (Lennox, 1955, Virology, 1:190) with 50 mg/l kanamycin. After incubation overnight at 37°C, recombinant individual clones were selected. Plasmid DNA was isolated from a transformant with the Qiaprep Spin Miniprep Kit (Product No. 27106, Qiagen, Hilden, Germany) in accordance with the manufacturer's instructions and cleaved with the restriction enzymes KpnI and XbaI to check the plasmid by subsequent agarose gel electrophoresis. The resulting plasmid was called pEC-XK99EsahHalex. It is shown in figure 2.

### Example 4

### Transformation of the strain DSM5715 with the plasmid pEC-XK99EsahHHalex

The strain DSM5715 was transformed with the plasmid pEC-XK99EsahHalex using the electroporation method described by Liebl et al., (FEMS Microbiology Letters, 53:299-303 (1989)). Selection of the transformants took place on LBHIS agar comprising 18.5 g/l brain-heart infusion broth, 0.5 M sorbitol, 5 g/l Bacto-tryptone, 2.5 g/l Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from a transformant by conventional methods (Peters-Wendisch et al., 1998, Microbiology, 144, 915 -927), cleaved with the restriction endonucleases KpnI and XbaI, and the plasmid was checked by subsequent agarose gel electrophoresis. The strain obtained was called DSM5715/pEC-XK99EsahHex1.

### Example 5

### Preparation of lysine

The C. glutamicum strain DSM5715/pEC-XK99EsahHalex obtained in example 4 was cultured in a nutrient medium suitable for the production of lysine and the lysine content in the culture supernatant was determined.

For this, the strain was first incubated on an agar plate with the corresponding antibiotic (brain-heart agar with kanamycin (25 mg/l)) for 24 hours at 33°C. Starting from this agar plate culture, a preculture was seeded (10 ml medium in a 100 ml conical flask). The complete medium CgIII was used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Kanamycin (25 mg/l) was added to this. The preculture was incubated for 16 hours at 33°C at 240 rpm on a shaking machine. A main culture was seeded from this preculture such that the initial OD (660nm) of the main culture was 0.1. Medium MM was used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 50 g/l |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing is carried out in a 10 ml volume in a 100 ml conical flask with baffles. Kanamycin (25 mg/l) was added. Culturing was carried out at 33°C and 80% atmospheric humidity.

After 48 hours, the OD was determined at a measurement wavelength of 660 nm with a Biomek 1000 (Beckmann Instruments GmbH, Munich). The amount of lysine formed was determined with an amino acid analyzer from Eppendorf-BioTronik (Hamburg, Germany) by ion exchange chromatography and post-column derivation with ninhydrin detection.

The result of the experiment is shown in table 1.

**Table 1**

| Strain | OD (660 nm) | Lysine HCl g/l |
|---|---|---|
| DSM5715 | 11.3 | 13.02 |
| DSM5715/pEC-XK99EsahHa1ex | 12 | 13.57 |

### Brief Description of the Figures:

- Figure 1:: Map of the plasmid pEC-XK99E.
- Figure 2:: Map of the plasmid pEC-XK99EsahHalex.

The abbreviations and designations used have the following meaning.
- Kan:: Kanamycin resistance gene aph(3')-IIa from Escherichia coli
- HindIII: Cleavage site of the restriction enzyme HindIII
- XbaI: Cleavage site of the restriction enzyme XbaI
- KpnI: Cleavage site of the restriction enzyme KpnI
- Ptrc: trc promoter
- T1: Termination region T1
- T2: Termination region T2
- per: Replication effector per
- rep: Replication region rep of the plasmid pGA1
- lacIq: lacIq repressor of the lac operon of Escherichia coli
- sahH: Cloned sahH gene

## Claims

1. Escherichia coli strain DH5αmcr/pEC-XK99sahHalex deposited as DSM 14316 at the Deutsche Sammlung für Mikroorganismen und Zellkulturen [German Collection of Microorganisms and Cell Cultures], Braunschweig, Germany.

2. A process for the preparation of L-lysine, which comprises carrying out the following steps:
a) fermentation of the coryneform bacteria which produce the desired L-amino acid and in which at least the sahH gene, comprising a nucleic acid sequence encoding an enzyme which is at least 90% identical to the amino acid sequence of SEQ ID NO: 2 and which is an adenosyl homocysteinase enzyme, or nucleotide sequences which code for it are over-expressed;
b) concentration of the L-amino acid in the medium or in the cells of the bacteria, and
c) isolation of the L-amino acid.

3. Process according to Claim 2, wherein said enzyme has the amino acid sequence of SEQ ID NO: 2.

4. Process according to Claim 2 or 3, wherein said nucleic acid has the nucleotide sequence of nucleotides 227 to 1720 of SEQ ID NO: 1.

5. A process according to claim 2, wherein a strain transformed with a plasmid vector is employed, and the plasmid vector carries the nucleotide sequence which codes for the sahH gene.

6. A process according to claim 2, wherein the expression of the polynucleotide(s) which code(s) for the sahH gene is over-expressed, so as to increase the activity or concentration of the corresponding enzyme by at least 10%.

7. A process according to claim 2, wherein coryneform bacteria in which at the same time one or more of the genes chosen from the group consisting of
7.1 the dapA gene which codes for dihydrodipicolinate synthase,
7.2 the gap gene which codes for glyceraldehyde 3-phosphate dehydrogenase,
7.3 the tpi gene which codes for triose phosphate isomerase,
7.4 the pgk gene which codes for 3-phosphoglycerate kinase,
7.5 the zwf gene which codes for glucose 6-phosphate dehydrogenase,
7.6 the pyc gene which codes for pyruvate carboxylase,
7.7 the mqo gene which codes for malate-quinone oxidoreductase,
7.8 the lysC gene which codes for a feed-back resistant aspartate kinase,
7.9 the lysE gene which codes for the protein for lysine export, and
7.10 the zwa1 gene which codes for the Zwa1 protein is or are over-expressed are fermented.

8. A process as claimed in claim 2, wherein for the preparation of L-amino acids, coryneform bacteria in which at the same time one or more of the genes chosen from the group consisting of
8.1 the pck gene which codes for phosphoenol pyruvate carboxykinase,
8.2 the pgi gene which codes for glucose 6-phosphate isomerase,
8.3 the poxB gene which codes for pyruvate oxidase,
8.4 the zwa2 gene which codes for the Zwa2 protein
is or are eliminated are fermented.

9. A process as claimed in one or more of claims 2 to 8, wherein microorganisms of the species Corynebacterium glutamicum are employed.

10. A process as claimed in claim 9, wherein the strain DSM5715/pEC-XK99EsahHalex is employed.

## Patentansprüche

1. Escherichia-coli-Stamm DH5αmcr/pEC-XK99sahHalex, hinterlegt unter DSM 14316 bei der Deutschen Sammlung für Mikroorganismen und Zellkulturen (DSMZ), Braunschweig.

2. Verfahren zur Herstellung von L-Lysin, bei dem man die folgenden Schritte durchführt:
a) Fermentation der coryneformen Bakterien, die die gewünschte L-Aminosäure produzieren und in denen wenigstens das sahH-Gen, umfassend eine für ein Enzym, das mindestens zu 90% mit der Aminosäuresequenz der SEQ ID NO: 2 identisch ist und bei dem es sich um ein Adenosylhomocysteinase-Enzym handelt, codierende Nukleinsäuresequenz, oder dafür codierende Nukleotidsequenzen überexprimiert werden;
b) Anreicherung der L-Aminosäure im Medium oder in den Zellen der Bakterien und
c) Isolierung der L-Aminosäure.

3. Verfahren gemäß Anspruch 2, wobei das Enzym die Aminosäuresequenz der SEQ ID NO: 2 aufweist.

4. Verfahren gemäß Anspruch 2 oder 3, wobei die Nukleinsäure die Nukleotidsequenz der Nukleotide 227 bis 1720 der SEQ ID NO: 1 aufweist.

5. Verfahren gemäß Anspruch 2, wobei ein mit einem Plasmidvektor transformierter Stamm eingesetzt wird und der Plasmidvektor die für das sahH-Gen codierende Nukleotidsequenz trägt.

6. Verfahren gemäß Anspruch 2, wobei die Expression des bzw. der für das sahH-Gen codierenden Polynukleotids bzw. Polynukleotide überexprimiert wird, so daß die Aktivität oder Konzentration des entsprechenden Enzyms um mindestens 10% erhöht wird.

7. Verfahren gemäß Anspruch 2, wobei coryneforme Bakterien fermentiert werden, in denen gleichzeitg eines oder mehrere der Gene, gewählt aus der Gruppe bestehend aus:
7.1 dem für Dihydrodipicolinat-Synthase codierenden Gen dapA,
7.2 dem für Glycerinaldehyd-3-phosphat-Dehydrogenase codierenden Gen gap,
7.3 dem für Triosephosphat-Isomerase codierenden Gen tpi,
7.4 dem für 3-Phosphoglycerat-Kinase codierenden Gen pgk,
7.5 dem für Glucose-6-phosphat-Dehydrogenase codierenden Gen zwf,
7.6 dem für Pyruvat-Carboxylase codierenden Gen pyc,
7.7 dem für Malat-Chinon-Oxidoreduktase codierenden Gen mgo,
7.8 dem für eine Feedback-resistente Aspartatkinase codierenden Gen lysC,
7.9 dem für das Protein für Lysin-Export codierenden Gen lysE und
7.10 dem für das Protein Zwa1 codierenden Gen zwa1,
überexprimiert wird oder werden.

8. Verfahren nach Anspruch 2, wobei zur Herstellung von L-Aminosäuren coryneforme Bakterien fermentiert werden, in denen gleichzeitg eines oder mehrere der Gene, gewählt aus der Gruppe bestehend aus:
8.1 dem für Phosphoenolpyruvat-Carboxykinase codierenden Gen pck,
8.2 dem für Glucose-6-phosphst-Isomerase codierenden Gen pgi,
8.3 dem für Pyruvatoxidase codierenden Gen poxB,
8.4 dem für das Protein Zwa2 codierenden Gen zwa2,
ausgeschaltet ist oder sind.

9. Verfahren nach einem oder mehreren der Ansprüche 2 bis 8, wobei Mikroorganismen der Spezies Corynebacterium glutamicum eingesetzt werden.

10. Verfahren nach Anspruch 9, wobei der Stamm DSM5715/pEC-XK99EsahHalex eingesetzt wird.

## Revendications

1. Souche de Escherichia coli DH5αmcr/pEC-XK99sahHalex déposée sous la référence DSM 14316 auprès de Deutsche Sammlung für Mikroorganismen und Zellkulturen [Collection Allemande de Microorganismes et de Cultures Cellulaires], Braunschweig, Allemagne.

2. Procédé de préparation de L-lysine, **caractérisé en ce qu'**il comprend la mise en oeuvre des étapes suivantes :
a) la fermentation des bactéries corynéformes qui produisent l'acide L-aminé recherché et dans lesquelles au moins le gène sahH, comprenant une séquence d'acides nucléiques codant pour une enzyme qui présente une identité d'au moins 90 % à la séquence d'acides aminés de SEQ ID NO : 2 et qui est une enzyme adénosyl homocystéinase, ou des' séquences nucléotidiques qui codent pour lui sont surexprimés ;
b) la concentration de l'acide L-aminé dans le milieu ou dans les cellules des bactéries ; et
c) l'isolement de l'acide L-aminé.

3. Procédé selon la revendication 2, **caractérisé en ce que** ladite enzyme présente la séquence d'acides aminés de SEQ ID NO : 2.

4. Procédé selon la revendication 2 ou 3, **caractérisé en ce que** ledit acide nucléique présente la séquence nucléotidique des nucléotides 227 à 1720 de SEQ ID NO : 1.

5. Procédé selon la revendication 2, **caractérisé en ce qu'**on utilise une souche transformée par un vecteur plasmidique et **en ce que** le vecteur plasmidique porte la séquence nucléotidique qui code pour le gène sahH.

6. Procédé selon la revendication 2, **caractérisé en ce que** l'expression du ou des polynucléotides codant pour le gène sahH est surexprimé de manière à augmenter l'activité ou la concentration de l'enzyme correspondante d'au moins 10 %.

7. Procédé selon la revendication 2, **caractérisé en ce qu'**on met en fermentation les bactéries corynéformes dans lesquelles est ou sont surexprimés conjointement un ou plusieurs des gènes choisis dans le groupe constitué du
7.1 gène dapA qui code pour la dihydrodipicolinate synthase,
7.2 gène gap qui code pour la glycéraldéhyde 3-phosphate déhydrogénase,
7.3 gène tpi qui code pour la triose phosphate isomérase,
7.4 gène pgk qui code pour la 3-phosphoglycérate kinase,
7.5 gène zwf qui code pour la glucose 6-phosphate déhydrogénase
7.6 gène pyc qui code pour la pyruvate carboxylase,
7.7 gène mqo qui code pour la malate-quinone oxydoréductase,
7.8 gène lysC qui code pour une aspartate kinase résistante à la rétroaction,
7.9 gène lysE qui code pour la protéine d'exportation de la lysine, et
7.10 gène zwa1 qui code pour la protéine Zwa1.

8. Procédé selon la revendication 2, **caractérisé en ce que** pour la préparation des acides L-aminés on met en fermentation des bactéries corynéformes dans lesquelles est ou sont éliminés conjointement un ou plusieurs des gènes choisis dans le groupe constitué du
8.1 gène pck qui code pour la phosphoénol pyruvate carboxykinase,
8.2 gène pgi qui code pour la glycose 6-phosphate isomérase,
8.3 gène poxB qui code pour la pyruvate oxydase,
8.4 gène zwa2 qui code pour la protéine Zwa2.

9. Procédé selon l'une ou plusieurs des revendications 2 à 8, **caractérisé en ce qu'**on utilise des microorganismes de l'espèce Corynebacterium glutamicum.

10. Procédé selon la revendication 9, **caractérisé en ce qu'**on utilise la souche DSM5715/pEC-XK99EsahHalex.
